Europäisches Patentamt

European Patent Office

Office européen des brevets

(11) Veröffentlichungsnummer: **0 371 156**
**A1**

(12) EUROPÄISCHE PATENTANMELDUNG

(21) Anmeldenummer: 88119825.3

(22) Anmeldetag: 28.11.88

(51) Int. Cl.5: **A61B 5/04**

(43) Veröffentlichungstag der Anmeldung:
06.06.90 Patentblatt 90/23

(84) Benannte Vertragsstaaten:
**DE FR GB IT NL**

(71) Anmelder: **Siemens Aktiengesellschaft**
**Wittelsbacherplatz 2**
**D-8000 München 2(DE)**

(72) Erfinder: **Naser, Georg, Dipl.-Ing.(FH)**
**Karlstrasse 10**
**D-8502 Zirndorf(DE)**
Erfinder: **Schneider, Siegfried, Dr.-Phys.**
**Kulmbacher Strasse 33**
**D-8520 Erlangen(DE)**

(54) **Anordnung zur Messung von im Körper eines Patienten örtlich und zeitabhängig auftretenden schwachen biomagnetischen Feldern.**

(57) Eine Mehrkanal-Meßanordnung zur Messung schwacher magnetischer Felder in lebenden Geweben und zur Lokalisierung der die Magnetfelder verursachenden Stromquellen aufgrund der Auswertung der von den einzelnen Sensoren der Meßanordnung gemessenen örtlichen magnetischen Werte, ist vertikal verstellbar an einer in Längs- und Querrichtung verstellbaren Patienten-Lagerungseinrichtung (2) befestigt, an deren Fußende zur Erzielung eines definierten Belastungszustandes eines Patienten (4) ein ausschließlich aus nichtmagnetischen Bauelementen bestehendes Fahrrad-Ergometer (9) derart montiert und ausgebildet ist, daß der Patient nach seiner Positionierung und Fixierung auf der Lagerungseinrichtung (2) das Ergometer betätigen kann.

FIG 1

EP 0 371 156 A1

# Anordnung zur Messung von im Körper eines Patienten örtlich und zeitabhängig auftretenden schwachen biomagnetischen Feldern

Die Erfindung betrifft eine Anordnung zur Messung von im Körper eines Patienten örtlich und zeitabhängig auftretenden schwachen biomagnetischen Feldern im Belastungs- und im Ruhezustand nach dem Oberbegriff des Patentanspruchs 1.

Es ist bekannt, den zeitlichen Verlauf der vom schlagenden Herzen ausgehenden elektrischen Impulse durch an bestimmten Stellen der Körperoberfläche angelegte Elektroden zu messen und in einem Elektrokardiogramm aufzuzeichnen. Diese Messungen erfolgen abwechselnd im Ruhezustand (Ruhe-EKG) und nach einer bestimmten, an einem Ergometer erbrachten und von diesem angezeigten körperlichen Leistung als Belastungs-EKG. Da die am Körper fixierten Elektroden über flexible Leitungen mit dem EKG-Meßgerät verbunden sind, sind diese Messungen unabhängig von der jeweiligen Lage und von Bewegungen des Körpers während der Messung.

Auf diese Art zustandegekommene Elektrokardiogramme sind zwar aussagekräftig bezüglich Amplitude und Frequenz der vom Herzen abgegebenen Impulse. Sie sind aber nicht geeignet, bestimmte, sich im Herzmuskel ereignende elektrische Vorgänge zu lokalisieren, d.h. eine Aussage über die räumliche Lage von elektrischen Vorgängen und deren zeitlichen Verlauf zu machen.

Es ist bekannt, zum Messen und Analysieren lokaler bioelektrischer Ströme in biologischen Gewebekomplexen, insbesondere des Gehirns und des Herzens, und deren Lokalisierung magnetische Meßmethoden anzuwenden und dabei Magnetoenzephalogramme (MEG) und Magnetokardiogramme (MKG) aufzunehmen.

Eine dazu geeignete, beispielsweise in der Zeitschrift "Bild der Wissenschaft", Heft 8. 1986, Seiten 76-83, beschriebene Einrichtung ist in der Lage, die äußerst schwachen biomagnetischen Signale, z.B. die in lebenden Gewebekomplexen durch gerichtete Stromflüsse entstehenden magnetischen Felder, deren Feldstärke in der Größenordnung von $10^{-12}$ T und darunter liegt, meßtechnisch zu erfassen. Diese Signale können von speziellen Sensoranordnungen nur dann gemessen werden, wenn Patient und Meßeinrichtung sorgfältig von äußeren magnetischen Feldern abgeschirmt sind und die Sensoranordnung zur genauen Lokalisation der Stromquelle im Gewebe mehrere sogenannte Gradiometer aufweist, die mit einer entsprechenden Anzahl von Squids (Super Conduction Quantum Interfierence Device) gekoppelt sind. Dabei müssen sowohl das Gradiometer wie das zugeordnete Squid in einem Kryostaten untergebracht sein, in dem eine Temperatur herrscht, bei dem die Squids und die Gradiometer supraleitend sind. Dabei steht der Fehler, mit dem die Stromquelle lokalisiert werden kann, in einem bestimmten Zusammenhang zu der Anzahl der Sensoren, d.h. der Meßpunkte; wie dies in der Zeitschrift "IEEE Transactions und Biomedical Engineering, Vol. 35 (8), August 1988, Seiten 573-575", insbesondere Absatz C in Verbindung mit Figur 5 erläutert ist. Um einen für die klinische Anwendung hinreichend kleinen Lokalisierungsfehler zu erzielen, müssen die magnetischen Signale, je nach dem Signal-zu-Rausch-Verhältnis, mit mindestens 10-12 Kanälen simultan gemessen werden.

Diese magnetischen Sensoren können schon wegen ihrer niedrigen Temperaturen nicht mehr, wie etwa EKG-Elektroden, am Körper selbst fixiert und über flexible Leitungen mit einem Zentralgerät verbunden werden. Vielmehr müssen diese Sensoren in einem gewissen Abstand zum Körper angeordnet werden. Dies hat zur Folge, daß sich der Körper während des Meßvorganges gegenüber den magnetischen Sensoren nicht räumlich verändern darf. Der Körper muß sich also gegenüber den Sensoren in einer fixierten Lage befinden, was insbesondere für die Messung nach Belastung des Patienten eine Rolle spielt. Es ist nämlich notwendig, die Belastungsmessung möglichst unverzüglich nach erfolgter Belastung vorzunehmen. Es besteht demzufolge keine Möglichkeit, den Patienten nach erfolgter ergometrischer Belastung einer Lagerungseinrichtung zuzuführen und dort zu fixieren, um dann anschließend das MKG aufzunehmen. Die dabei vergehende Zeit wäre zu lang, um noch eine aussagekräftige Belastungsmessung vornehmen zu können.

Es ist nun grundsätzlich bereits bekannt, einen bettlägerigen Patienten durch einen am Fußende fixierten Mechanismus Belastungen zu unterziehen, um etwa nach Operationen Embolien entgegenzuwirken. Dabei handelt es sich aber nicht um Ergometer zum Zwecke der Messung etwa von Belastungs-EKGs oder Belastungs-MKGs, sondern um reine Trainingsgeräte.

Der Erfindung liegt die Aufgabe zugrunde, eine Anordnung zu finden, mit der es möglich ist, auch Belastungs-Magnetokardiogramme aufzunehmen, die unverzüglich nach einer definierten Belastung des Patienten, also ohne dessen Umlagerung, gemacht werden können, ohne daß Fremdmagnetfeldeinflüsse oder Verzerrungen des biomagnetischen Feldes zu befürchten sind.

Diese Aufgabe wird durch die im Patentanspruch 1 angegebene Erfindung gelöst. Dadurch ist erreicht, daß die Lagerungseinrichtung für den Patienten, das Ergometer und das MKG-System eine Einheit bilden, die es ermöglicht, unmittelbar nach einer definierten Belastung ein MKG des Patienten aufnehmen zu können.

Aufgrund eines Ausführungsbeispiels wird die Erfindung im folgenden erläutert. Darin zeigen:

Fig. 1 eine Mehrkanal-Meßanordnung, verbunden mit einer Patienten-Lagerungseinrichtung mit integriertem Fahrrad-Ergometer; und

Fig. 2 den prinzipiellen Aufbau eines für das Zusammenwirken mit der Meßanordnung geeigneten Fahrrad-Ergometers.

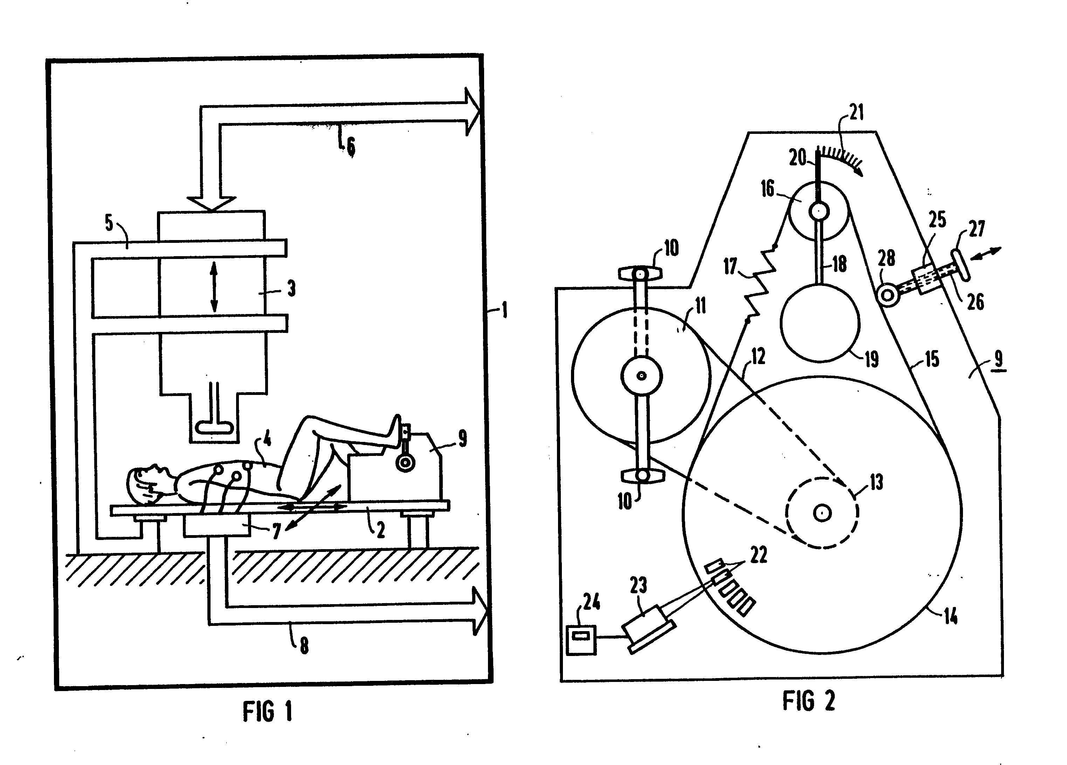

Die in Fig. 1 dargestellte magnetische Abschirmkammer 1 beinhaltet eine Patienten-Lagerungseinrichtung 2 sowie eine Meßanordnung 3 zur Messung der von einem auf der Patienten-Lagerungseinrichtung 2 liegenden Patienten 4 ausgehenden biomagnetischen Felder. Die Meßanordnung ist an einem mit der Patienten-Lagerungseinrichtung mechanisch verbundenen Halterungsgestell 5 in vertikaler Richtung verstellbar befestigt. Die Meßanordnung 3 ist über einen Datenbus 6 mit einem nicht dargestellten System für die Auswertung und Darstellung der Daten als Magnetokardiogramm (MKG) verbunden. Zusätzlich kann der Meßanordnung 3 ein EKG-Gerät 7 beigestellt werden, so daß neben den MKG-Daten auch EKG-Daten zur Verfügung stehen und über den EKG-Datenbus 8 an ein nicht dargestelltes EKG-Aufzeichnungsgerät gelangen. Am Fußende der Patienten-Lagerungseinrichtung 2 ist ein Fahrrad-Ergometer 9 derart befestigt, daß der Patient 4 dieses im liegenden Zustand betätigen kann.

In Fig. 2 ist das Fahrrad-Ergometer 9 schnittbildlich dargestellt. Der prinzipielle Aufbau ist bekannt. Ein von Pedalen 10 betätigbares Antriebsrad 11 ist über einen Riementrieb 12 mit dem Ritzel 13 eines Schwungrades 14 verbunden, dessen Umfang über einen bestimmten Winkel von einem Bremsband 15 umschlungen ist. Dieses Bremsband ist über den Außenmantel einer Pendelrolle 16 geführt und wird über eine Zugfeder 17 auf Zug gehalten. Die Pendelrolle 16 ist über das Pendel 18 mit einem Pendelgewicht 19 verbunden, welches in Abhängigkeit von den an der Pendelrolle 16 auftretenden tangentialen Reibungskräften bei einer Bewegung des Schwungrades 14 entgegengesetzt zur Wirkrichtung der Tangentialkraft um einen von dieser abhängigen Winkel um den Drehpunkt der Pendelrolle 16 bewegt wird. Dabei ist der Pendelwinkel ein Maß für die auf das Schwungrad 14 ausgeübte Leistung. Dazu ist das Pendel 18 mit einem dieses über den Außendurchmesser der Pendelrolle 16 hinaus verlängernden Anzeigestab 20 versehen, dessen Spitze auf eine in Watt geeichte Anzeigeskala 21 weist.

Das Schwungrad 14 ist mit zahnkranzartig angeordneten Reflektoren 22 versehen, auf die der Lichtstrahl eines optoelektronischen Drehzahlmessers 23 gerichtet ist, der aufgrund der Anzahl der pro Zeiteinheit reflektierten Lichtimpulse die Drehzahl pro Zeiteinheit des Schwungrades ermittelt und einem Anzeigegerät 24 zuführt.

Um die Größe der jeweils gewünschten Belastung des Patienten in einem weiten Bereich einstellen zu können, ist ein Belastungs-Einstellmittel vorgesehen, welches auf das Bremsband 15 in der Weise einwirkt, daß dessen Spannkraft durch seitliches Eindrücken von einem Minimalwert auf einen Maximalwert stufenlos erhöht werden kann. Dazu dient eine in einer Gewindebuchse 25 drehbar gelagerte Gewindespindel 26, die außen mit einem Einstellrad 27 versehen ist, und bandseitig eine Andruckrolle 28 besitzt. Durch Betätigen des Handrades kann nun die Spannkraft des Bremsbandes 15 und damit die gewünschte Leistung gewählt werden.

Wichtig für die Funktion der Meßanordnung ist es nun, daß das von den bioelektrischen Stromquellen jeweils erzeugte biomagnetische Feld, welches von der Meßanordnung 3 in seiner räumlichen Lage und seinem zeitlichen Ablauf ermittelt werden soll, nicht durch magnetische Fremdfelder gestört oder durch in seiner Nähe befindliche ferromagnetische Materialien verzerrt wird. Ein übliches Ergometer besteht aber im wesentlichen aus ferromagnetischen Materialien. Ein wesentlicher Gesichtspunkt der Erfindung besteht nun darin, das Ergometer ausschließlich aus unmagnetischen Werkstoffen aufzubauen. Als solche kommen Aluminium, Bronze, organische Kunststoffe und unmagnetischer Stahl in Betracht. Auch die Meßmittel für die Leistung und die Drehzahl dürfen keine elektromagnetischen Felder erzeugen.

## Ansprüche

1. Mehrkanal-Meßanordnung zur Messung schwacher magnetischer Felder in lebenden Geweben und zur Lokalisierung der die Magnetfelder verursachenden Stromquellen aufgrund der Auswertung der von den einzelnen Sensoren der Meßanordnung gemessenen örtlichen magnetischen Werte und deren zeitlichen Verlauf im Ruhezustand und im Belastungszustand des Patienten, **dadurch gekennzeichnet,** daß zur Erzielung eines definierten Belastungszustandes des Patienten ein Ergometer (9) fest am Fußende einer in Längs- und Querrichtung verstellbaren Patienten-Lagerungseinrichtung (2) derart montiert und ausgebildet ist, daß der Patient (4) nach seiner Positionierung und Fixierung auf der Lagerungseinrichtung (2) das Ergometer (9) betätigen kann, daß die Mehrkanal-Meßanordnung (3) an einem mit der Lagerungseinrichtung (2) verbundenen Halterungsgestell (5) vertikal zu dieser verstellbar befestigt ist und daß das Ergometer (9) ausschließlich aus nichtmagnetischen und kein Magnetfeld erzeugenden Bauelementen (10-28) besteht.

2. Anordnung nach Anspruch 1, **dadurch gekennzeichnet,** daß als Ergometer (9) ein Fahrrad-Ergometer benutzt ist, das zur Erzielung eines definierten Drehmomentes eine einstellbare, auf ein Schwungrad (14) wirkende Bandbremse (15, 17) sowie eine mechanische Meßanordnung (16, 18, 19, 20, 21) für die aufgebrachte mechanische Leistung und einen auf das Schwungrad (14) wirkenden optoelektronischen Drehzahlmesser (22, 23, 24) mit optischer Anzeige besitzt.

3. Anordnung nach Anspruch 1 oder 2, **dadurch gekennzeichnet,** daß der Meßanordnung zusätzlich ein EKG-Gerät (7, 8) beigestellt ist.

4. Anordnung nach einem der vorhergehenden Ansprüche, **dadurch gekennzeichnet,** daß das Ergometer (9) mit einfachen Hilfsmitteln lösbar und in definierter Lage wieder anbringbar an der Patienten-Lagerungseinrichtung (2) befestigt ist.

FIG 1

FIG 2

EP 0 371 156 A1

88 P 3 5 1 7 E

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | IEEE TRANSACTIONS ON BIOMEDICAL ENGINEERING Band 35, Nr. 9, September 1988, Seiten 662-669, New York, USA; R. MANIEWSKI et al.: "Magnetic Measurements of Cardiac Mechanical Activity" * Seite 664, rechte Spalte, Zeile 24 - Seite 665, rechte Spalte, Zeile 9; Figur 3 * --- | 1,3 | A 61 B 5/04 |
| A | EP-A-0 277 283 (SIEMENS AG) * Spalte 4, Zeilen 27-30; Figur * --- | 1 | |
| A | BIOMEDIZINISCHE TECHNIK Band 25, Nr. 11, November 1980, Seiten 285-292, West-Berlin, BRD; R. HINSEN et al.: "EKG-Mapping unter Belastung zur Diagnose der koronaren Herzerkrankung" * Seite 286, linke Spalte, Zeile 39 - rechte Spalte, Zeile 11; Figur 1 * --- | 1,4 | |
| A | US-A-3 767 195 (K. P. DIMICK) * Spalte 2, Zeile 65 - Spalte 3, Zeile 22; Spalte 5, Zeilen 11-20; Figuren 1,2 * ----- | 2 | RECHERCHIERTE SACHGEBIETE (Int. Cl.5) A 61 B 5/00 |

Der vorliegende Recherchenbericht wurde für alle Patentansprüche erstellt

| Recherchenort | Abschlußdatum der Recherche | Prüfer |
|---|---|---|
| BERLIN | 11-07-1989 | WEIHS J.A. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

......................................................................
& : Mitglied der gleichen Patentfamilie, übereinstimmendes Dokument

EPO FORM 1503 03.82 (P0403)